(19) Europäisches Patentamt / European Patent Office / Office européen des brevets

(11) **EP 3 132 850 A1**

(12) **EUROPEAN PATENT APPLICATION**

(43) Date of publication:
**22.02.2017 Bulletin 2017/08**

(51) Int Cl.:
***B01L 3/00*** *(2006.01)*

(21) Application number: **15181556.0**

(22) Date of filing: **19.08.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(71) Applicant: **International Iberian Nanotechnology Laboratory**
**4715-330 Braga (PT)**

(72) Inventors:
• **Bi, Hongyan**
**4715-330 Braga (PT)**
• **Freitas, Paulo**
**4715-330 Braga (PT)**

(74) Representative: **Awapatent AB**
**P.O. Box 1066**
**251 10 Helsingborg (SE)**

(54) **SYSTEM AND METHOD FOR ANALYSIS OF A TARGET MOLECULE IN A COMPLEX MATRIX SAMPLE**

(57) The present invention relates to a system (1) for analysis of a target molecule in complex matrix sample, the system comprising: a chip (2) comprising a reaction flow channel (4) configured to receive the complex matrix sample, wherein the reaction flow channel is coated with an enzyme (12) configured to chemically change the target molecule; a flow generator (6), configured to provide a flow of the complex matrix sample through the reaction flow channel; and an absorbance detector (8) configured to determine the absorbance of the complex matrix sample after having passed through the reaction flow channel. The invention further relates to a method for analysis of a target molecule in a complex matrix sample.

Fig. 1

**Description**

Technical Field

The present invention relates to a system for analysis of a target molecule in a complex matrix sample including, but not limited to, food matrices like beverages, tablets (dietary supplement products), and fruits; and human body fluids like plasma and serum.

Background

Nutrient and flavour components of food, for example protein, polysaccharide, antioxidant, and ionic minerals, effect the taste of the food and providing the food nutritonal values. Other food ingredients such as antioxidants, toxins, allergens and contaminants effect human health. Therefore food analysis is of importance.

Due to an awareness of how the component composition of food affect taste and health, specific food analysis is becoming increasingly important.

It is a problem with analysis of food that it frequently involves analysis of complex matrix samples.

Different from a standard solution where the solvent and solute are usually known. In a complex matrix sample, the target molecule can be assigned, though the other components are unknown. The other components of the sample is called a matrix. The matrix is normally formed by many different components; hence the matrix is complex. Take blood serum as an example, the ascorbic acid in the serum can be assigned as the target molecule, while the proteins and other components, like caffeine, in the blood are then the complex matrix. With the same serum sample, if assigning caffeine as the target molecule, the other components including ascorbic acid are then the complex matrix.

The analysis to a certain ingredient in biological samples within a complex matrix background can be a challenge, but the determination of it can be important. For example, abnormal concentration of some small organic molecules, like ascorbic acid, in blood can be associated with a disease. The background complex matrix can interfer the measurement of ascorbic acid. For example, coffeine and proteins in serum can affect the detection of ascorbic acid when directly using UV/Vis spectroscopy for ascorbic acid determination in serum. Mass Spectrometry, chromatography, eletrophoresis and electrochemistry are methods and techniques that may be used to quantify levels of various components in complex matrix samples. These techniques are often time-consuming and usually require pretreatment or separation procedures.

Ultraviolet, UV, and/or visual, Vis, spectroscopy may be capable of providing rapid and quantitative analysis of samples. However, it is inherent to the method that molecules having similar spectroscopic properties as the compound of interest may interfere with the analysing process. For example, an interfering molecule may also have UV/Vis absorption as the compound of interest at the same wavelength.

Thus, there is a need for analytical systems capable of efficiently handling samples within a complex matrix background, i.e. handling complex matrix samples.

Summary of invention

An object of the present invention is to provide a system for analysis of a complex matrix sample, without disadvantages of prior art.

Another object is to provide a system for analysis of a target molecule in complex matrix sample, which results in efficient analysis.

These and other objects of the invention are at least partly met by the invention as defined in the independent claims. Preferred embodiments are set out in the dependent claims.

The systems and methods according to aspects described herein provides for analysis of target molecules also when present in complex matrix samples, the complex matrix samples may be considered as complex for example in that the sample comprises considerable amount of contaminants.

According to a first aspect there is provided a system for analysis of a target molecule in a complex matrix sample. The system comprising: a chip comprising a reaction flow channel configured to receive the complex matrix sample, wherein the reaction flow channel is coated with enzyme configured to chemically change the target molecule; a flow generator, configured to provide a flow of the complex matrix sample through the reaction flow channel; and an absorbance detector configured to determine the absorbance of the complex matrix sample after having passed through the reaction flow channel.

The chip is a suitable format for providing rapid and efficient analysis.

The reaction flow channel being coated with enzyme enables efficient reactions between the enzyme and the target molecule and thereby chemically changing the target molecule.

Chemical change of the target molecule allows for detecting a change in absorbance, and thus for determining presence of target molecules in the complex matrix sample.

The flow generator is an efficient means for providing flow through the reaction flow channel.

The absorbance detector is an efficient means for determining the absorbance of the complex matrix sample, and/or compounds present in the complex matrix sample.

The reaction flow channel may be a micro fluidic flow channel having a cross sectional area taken perpendicular to a flow direction of the complex matrix sample through the reaction flow channel below 100 000 $\mu m^2$.

The reaction flow channel may be a micro fluidic flow channel having a cross sectional area taken perpendicular to a flow direction of the complex matrix sample through the reaction flow channel in the range of 25 $\mu m^2$ to 100 000 $\mu m^2$.

Such cross sectional areas provides for efficient analysis.

The length of the reaction flow channel may be 1 to 100 cm.

Such a length provides for efficient analysis.

The reaction flow channel may be coated with the enzyme by physisorption. Thus efficient coating is provided.

The reaction flow channel may be coated with the enzyme by sol-gel encapsulation, or by chemical immobilization.

Thus, a durable and stable coating may be provided.

The reaction flow channel may be chemically modified with the enzyme.

Thus, a durable and stable coating may be provided.

It is realized that any suitable method for immobilizing the enzyme in the reaction flow channel may be used.

The absorbance detector may be an absorbance spectrophotometer.

Thus an efficient means for detecting a change in the presence of target molecule, such as a target molecule concentration, is provided. Thus, a presence of target molecules in the complex matrix sample may be detected and/or quantified.

The absorbance spectrophotometer may be configured to determine absorbance in the visible and/or ultraviolet and/or near-infrared, NIR, wavelength range of electromagnetic radiation.

Thus an efficient means for detecting a change in the presence of target molecule, such as a change in target molecule concentration, is provided.

The chip may be manufactured from, comprise, or essentially consist of a material selected from the list consisting of silicon, glass, paper, and polymers, such as SU-8, PDMS, PC, PET, PE/PET, polyimide, and PMMA, and combinations thereof.

The absorbance detector may further be configured to detect an additional absorbance of complex matrix sample which has not passed the reaction flow channel, or which has passed the reaction flow channel to a lesser extent as compared to the absorbance of the complex matrix sample having passed the reaction flow channel.

The complex matrix sample may be a food sample, dietary supplement products, and/or bio-samples like serum.

According to a second aspect, there is provided a method for analysis of a target molecule in a complex matrix sample, the method comprising: contacting the complex matrix sample and an enzyme configured to chemically change the target molecule; determining a first absorbance of the complex matrix sample, which complex matrix sample has been subjected to the contacting according to a first degree; determining a second absorbance of the complex matrix sample, which complex matrix sample has been subjected to the contacting according to a second degree, wherein the second degree of contacting is larger than the first degree of contacting; and comparing the first absorbance and the second absorbance.

By subjecting the complex matrix sample for the contacting according to a first degree refers to subjecting the complex matrix sample for the contacting to a lesser degree than subjecting the complex matrix sample for the contacting according to the second degree. For example, subjecting the complex matrix sample for the contacting according to a first degree may refer to not subjecting the complex matrix sample for the contacting at all. Alternatively, subjecting the complex matrix sample for the contacting according to a first degree may refer to subjecting the complex matrix sample for the contacting only within a first portion of the reaction flow channel, which first portion of the reaction flow channel is smaller than a second portion of the reaction flow channel used for subjecting the complex matrix sample for the contacting according to the second degree. The second portion of the reaction flow channel may refer to the whole reaction flow channel.

The comparison between the first absorbance and the second absorbance enables efficient qualitative and/or quantitative determination of the target molecule.

The method may further comprise: coating a contacting surface with the enzyme prior to the contacting.

Thus efficient immobilization of the enzyme is realized.

The method may further comprise: quantifying the target molecule using the comparison.

The chemical change of the target molecules may involve a consumption of the target molecule. Thus, efficient qualitative and/or quantitative determination of the target molecule is realized.

The first absorbance and the second absorbance may be within the visible and/or ultraviolet and/or NIR wavelength range of electromagnetic radiation.

According to a third aspect there is provided the method according to the second aspect using the system according to the first aspect.

A further scope of applicability of the present invention will become apparent from the detailed description given below. However, it should be understood that the detailed description and specific examples, while indicating preferred embodiments of the invention, are given by way of illustration only, since various changes and modifications within the scope of the invention will become apparent to those skilled in the art from this detailed description.

Hence, it is to be understood that this invention is not limited to the particular component parts of the device described or steps of the methods described as such device and method may vary. It is also to be understood that the terminology used herein is for purpose of describing particular embodiments only, and is not intended to be limiting. It must be noted that, as used in the specification and the appended claim, the articles "a," "an," "the," and "said" are intended to mean that there are one or more of the elements unless the context clearly dictates otherwise. Thus, for example, reference to "a unit" or "the unit" may include several devices, and the like. Furthermore, the words "comprising", "including", "containing" and similar wordings does not exclude other elements or steps.

Brief Description of the Drawings

The above and other aspects of the present invention will now be described in more detail, with reference to appended drawings showing embodiments of the invention. The figures should not be considered limiting the invention to the specific embodiment; instead they are used for explaining and understanding the invention.

As illustrated in the figures, the sizes of layers and regions are exaggerated for illustrative purposes and, thus, are provided to illustrate the general structures of embodiments of the present invention. Like reference numerals refer to like elements throughout.

Fig. 1 illustrates schematically a top view of the system according to an embodiment.

Fig. 2 illustrates schematically a cross-sectional view, taken along the flow direction, of a part of the reaction flow channel.

Fig. 3 illustrates schematically a top view of the system according to an embodiment.

Fig. 4 illustrates schematically a top view of the system according to an embodiment.

Fig. 5 illustrates a method according to an embodiment.

Fig. 6 illustrates a calibration curve according to an example.

Figs 7a and 7b illustrate absorbance chromatograms according to an example.

Detailed description

The present invention will now be described more fully hereinafter with reference to the accompanying drawings, in which currently preferred embodiments of the invention are shown. This invention may, however, be embodied in many different forms and should not be construed as limited to the embodiments set forth herein; rather, these embodiments are provided for thoroughness and completeness, and to fully convey the scope of the invention to the skilled person.

According to the embodiments, a target molecule may be allowed to react with an enzyme inside a chip, whereby a chemical change specific to the target molecule may be obtained, while interfering compounds in the sample are unaffected. Thereby a target molecule in the complex matrix sample may specifically be detected and/or quantified, for example by determining a change in a detection signal which changes with the concentration or presence of the target molecule. By comparing a detection signal obtained from the complex matrix sample which has not undergone contacting with the enzyme with a detection signal obtained from the complex matrix sample which has undergone contacting with the enzyme, the target molecules may be qualitatively and/or quantitatively analysed even in the presence of a complex sample matrix having identical or similar absorbance properties as the target molecule.

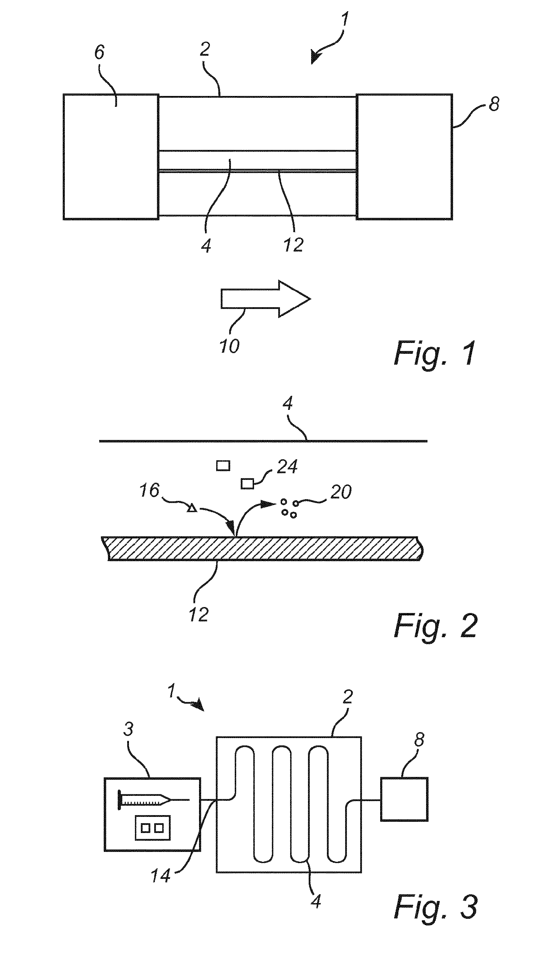

With reference to Fig. 1, a system 1 for analysis of a target molecule in complex matrix sample will now be described. The system 1 comprises a chip 2 comprising a reaction flow channel 4 configured to receive the complex matrix sample, wherein the reaction flow channel 4 is coated with an enzyme 12 configured to chemically change the target molecule; a flow generator 6, configured to provide a flow of the complex matrix sample through the reaction flow channel 4; and an absorbance detector 8 configured to determine the absorbance of the complex matrix sample after having passed through the reaction flow channel 4. The direction of the flow through the reaction flow channel is schematically illustrated by arrow 10.

Enzymes may be selected to be specifically active towards a specific target molecule, and, thus, the absorbance of the complex matrix sample after having passed through the reaction flow channel 4 may provide information on presence of the target molecule in the complex matrix sample. The enzyme may catalyze the reaction of target molecules.

Determining the presence of target molecules in the complex matrix sample may efficiently be realized, for example, by comparing a detector signal obtained before and after contacting between the complex matrix sample and the enzyme.

It is realized that a plurality of target molecules may be analysed with the system. The target molecules may be identical, although it is realized that target molecules may be in a plurality of forms having such similarities such that the enzyme chemically may change the target molecules. Thus, target molecule may refer to a single type of compound, or to a group of compounds sensitive to the enzyme.

The enzyme is configured to chemically change the target molecule. This may involve chemical reactions of the target molecule, such that the target molecule is reacted under formation of reaction products. The reaction products have absorbance properties being different compared to the unreacted target molecule.

The reaction flow channel may be coated with two or more enzymes, each enzyme being active to chemically change one different target molecule respectively. Thus, a plurality of different target molecules may be analysed with the system.

The complex matrix sample may be food or food derived samples comprising the target molecule. The complex matrix sample may be food or food derived samples suitably mixed with solvents or buffers, and the complex matrix sample may comprise additives.

The complex matrix sample may be bio-samples comprising the target molecule.

The chip 2 may have any suitable shape or form, for example a flat and thin shape. The chip 2 may be manufactured from, essentially consist of, or comprise a material selected from the list consisting of silicon, glass, paper, and polymers, such as PDMS, PC, PET, PE/PET, polyimide, and PMMA, and combinations thereof. According to an embodiment, the chip 2 is made of the polymer material polydimethylsiloxane (PDMS) with a glass lamination.

The reaction flow channel 4 of the chip 2 is configured to receive the complex matrix sample. The reaction flow channel 4 may have an inlet-opening or portion 14 for receiving of complex matrix sample. The reaction flow channel 4 is coated with an enzyme configured to chemically change the target molecule, as discussed with reference to figure 3 herein. The reaction flow channel 4 of this embodiment is a micro-fluidic flow channel having a cross sectional area taken perpendicular to a flow direction of the complex matrix complex matrix sample through the reaction flow channel of 25 to 100 000 $\mu m^2$. The length of the reaction flow channel may be, for example, 1 to 100 cm.

The reaction flow channel 4 may be created using standard manufacturing techniques. The reaction flow channel 4 may be made by using soft lithography. The reaction flow channel 4 may have various suitable shapes and geometries. For example, the geometry perpendicular to the reaction flow channel 4 may be rectangular, oval or circular shaped. The reaction flow channel 4 may further have a serpentine or undulating elongation in the chip 2 thereby allowing for a compact design of the chip 2.

Allthough not illustrated, the reaction flow channel 4 may comprise a plurality of separated portions or parts coated with an enzyme. Thus, a plurality of portions or parts coated with enzyme may be separated by parts or portions of the reaction flow channel 4 being uncoated with enzyme.

The flow generator 6 configured to provide a flow of the complex matrix sample through the reaction flow channel 4 may be connected to the reaction flow channel in different suitable ways. The flow generator 6 may be directly or indirectly connected to the reaction flow channel 6. For example, the flow generator may be indirectly connected by means of one or more of tubings, channels or capillaries, or combinations thereof. The flow channel may be connected to the inlet opening or portion of the reaction flow channel 4. The flow generator 6 may be a pump, such as a syringe pump, a peristaltic pump or a pressure pump. The flow generator 6 may be operated manually or energized. The flow generator may also be a capillary or any other narrow channel or passage arranged in connection with the reaction flow channel such that liquid is introduced by means of capillary action.

The flow generator 6 may provide a flow such as a peristaltic flow, a continuous or a periodical flow, or combinations thereof. The flow may be provided at different flow rates. The flow may be turned on and off during different time intervalls. The flow generator 6 may further be connected to an outlet opening of the reaction flow channel 6 and use suction force to generate flow in the flow channel 4.

The enzyme 12 may be coated on the inner surface of the reaction flow channel 4. The coating may be done by physisorption. Other coating methods may be used, such as sol-gel encapsulation or chemically modyfying the reaction flow channel 4. Any suitable method for coating the reaction flow channel with the enzyme may be used. The reaction flow channel 4 may, for example, have one, two or more, or all of the walls coated with enzyme. It is realized that the reaction flow channel 4 may comprise coating structures being coated with the enzyme. For example, the reaction flow channel 4 may have beads or microstructures being coated with the enzyme.

The absorbance detector may be a UV/vis absorbance detector. The absorbance detector may be a broadband absorbance detector or an absorbance detector with a narrow wavelength detection window. The absorbance detector is configured to determine the absorbance of the complex matrix sample after having passed through the reaction flow channel. This may be done by measuring the absorbance directly on the chip, through the flow channel, or off the chip. Off the chip measurements may be done by allowing the complex matrix sample to flow to the detector via suitable flow channels, tubing, or capillaries, or any other suitable means. It is realized that the sample alternatively may be sampled or collected after having passed the reaction flow channel, and thereafter injected into a detector which is not directly connected to the chip or reaction flow channel. Qualitative or quantitative analysis of the target molecule may be made by comparing the absorbance obtained after the sample has passed the reaction flow channel with an additional absorbance value obtained from the complex matrix sample which has not undergone reaction with the enzyme, or having undergone less reaction with the enzyme. The absorbance detector may suitably be configured to measure absorbance of a wavelength window where the target molecule absorbs. Thereby, the consumption of target molecules in the reaction flow channel will lead to a decrease in absorbance, which allows for detection of the target molecule in the complex matrix sample, even for such cases that contaminants of the complex matrix sample absorbs in the same wavelength window where the target molecule absorbs.

The absorbance detector may further be configured to determine an additional absorbance of the complex matrix sample which has not passed through the reaction flow channel 4. The additional absorbance may be obtained from the complex matrix sample before having entered or before having passed through the reaction flow channel. Alternatively or in combination, the additional absorbance may be obtained from a portion of the complex matrix sample which is not allowed to pass through the reaction flow channel. For example, the absorbance detector may be configured to measure the absorbance of the complex matrix sample on the chip before the complex matrix sample has been forwarded to the reaction flow channel. Alternatively, the flow of complex matrix sample may, for example, be split upstream of the reaction flow channel, whereby absorbance is measured on a split of the flow which has not passed the reaction flow channel. According to another example, a fraction of the complex matrix sample may be obtained, such as from a vessel holding the complex matrix sample, and presented to the absorbance detector. It is realized that the absorbance detector may contain a plurality of units able to measure absorbance of a sample.

The enzyme 12 being configured to chemically change the target molecule will now be further discussed with reference to Fig. 2. The chemical change involves reactions between the target molecule 16 and the enzyme 12 under formation of reaction product 20. The enzyme 12 may be selected such that the target molecule 16 have a suitable level of affinity to the enzyme 12. The reaction product 20 may have absorbance properties being different compared with the target molecule 16 for providing efficient analysis.

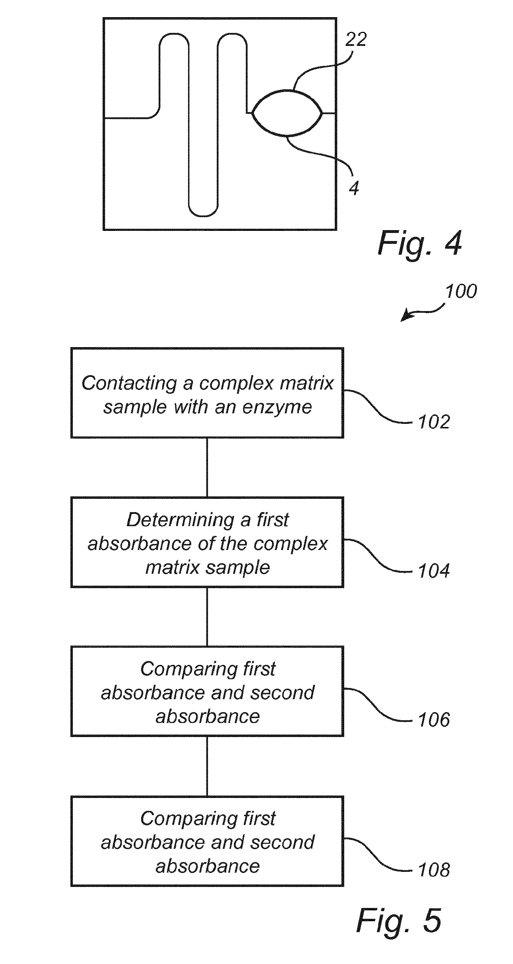

An embodiment of the system will now be discussed with reference to Fig. 3 illustrating a system 1 according to an embodiment. Target molecules of a complex matrix sample is analyzed by the system 1 by using an absorbance detector 8 set to measure the absorbance in a narrow wavelength window in which the target molecules are absorbing, but in which the reaction product of the contacting between the enzyme and the target molecules do not absorb or absorb differently from the target molecules. The absorbance detector 8 may be a UV/Vis spectrophotometer. In this example, the complex matrix sample is liquid food stuff, or can be prepared as liquid. The complex matrix sample is injected directly into the detector, without having passed the reaction flow channel 4 of the chip 2, thereby obtaining a first absorbance. In this example this is performed by manually injecting the complex matrix sample by means of a syringe into the detector 8. Further, the complex matrix sample is injected into the reaction flow channel 4 through an inlet opening or portion 14. The reaction flow channel 4 is coated with an enzyme reactive towards the target molecules. The injection of sample and pumping of the sample through the reaction flow channel is realized by means of the flow generator 3 in this example being a syringe pump. After having passed the reaction flow channel 4, the complex matrix sample is pumped further to the detector 8, wherein a second absorbance measurement is obtained. By comparing the second absorbance measuring and the first absorbance measuring, it is determined that the second absorbance is lower than the first resulting from the consumption of taget molecules due to contacting with the enzyme. The difference may be used to determine that target molecules were present in the complex sample matrix, and the difference may be used to quantify the target molecules. Thus, for example, the first absorbance may be obtained using an absorbance detector 8 positioned upstream the reaction flow channel 4. The first absorbance may alternatively be obtained in the reaction flow channel 4 using the absorbance detector 8, before the enzyme has been immobilized in the reaction flow channel 4. Alternatively, the first absorbance may be obtained in a channel 22 parallel to the reaction channel 4, wherein the paralell channel 22 does not contain the enzyme 4, as illustrated in Fig. 4.

The second absorbance is obtained of the complex matrix sample after the complex matrix sample has passed through the reaction flow channel 4.

The first and second absorbances may thereafter be compared.

The complex matrix sample may comprise interference molecules 24. The interference molecules 24 may have the same or very close absorbance properties as the target molecules 16. The interference molecules 24 may therefore have an effect on the first and second absorbances. However, since the enzyme 12 has a specificity for the target molecules 16, the interference molecules 24 are not reacting with the enzyme and the difference between the first and second measurement is dependant on the presence of the target molecules 16.

The method for analysis of a target molecule in a complex matrix sample according to an embodiment will now be discussed with reference to Fig. 5. The complex matrix sample may be, for example, a food or food derived samples, or other samples comprising the target molecule. The complex matrix sample may further comprise interference molecules. The method 100 comprises contacting 102 the complex matrix sample and an enzyme configured to chemically change the target molecule. The method 100 further comprises determining 104 a first absorbance of the complex matrix sample, which complex matrix sample has been subjected to the contacting according to a first degree; determining 106 a second absorbance of the complex matrix sample, which complex matrix sample has been subjected to the contacting according to a second degree, wherein the second degree of contacting is larger than the first degree of contacting; and comparing 108 the first absorbance and the second absorbance.

By subjecting the complex matrix sample for the contacting according to a first degree refers to subjecting the complex matrix sample for the contacting to a lesser degree than subjecting the complex matrix sample for the contacting according to the second degree. For example, subjecting the complex matrix sample for the contacting according to a first degree may refer to not subjecting the complex matrix sample for the contacting at all. Alternatively, subjecting the complex matrix sample for the contacting according to a first degree may refer to subjecting the complex matrix sample for the contacting only within a first portion of the reaction flow channel, which first portion of the reaction flow channel is smaller than a second portion of the reaction flow channel used for subjecting the complex matrix sample for the contacting according to the second degree. The second portion of the reaction flow channel may refer to the whole reaction flow channel.

It is realized that the determining 104 a first absorbance of the complex matrix sample may be performed before the contacting 102, and that the determining 106 a second absorbance of the complex matrix sample may be performed after the complex matrix sample has been contacted to the enzyme.

Example: Analysis of L-ascorbic acid

Bellow follows a non-limiting example of the system 1 when in use. A person skilled in the art realizes that among others, the materials and the chemical compounds being used may be changed as well as the size, shape and form of chip 2 and flow channel 4.

L-ascorbic acid (AA) is used as an example of a target molecule 16. AA shows a characteristic and maximum UV/vis absorbance level at 266 nm. A higher level of absorbance in the measurement relates to a higher concentration of AA. However, in a complex matrix sample, such as a food derived sample, there are many compounds that may interfere with the quantification of AA. For example, peptides, alcohols, caffeine, and color additives may also have absorbance at around 266nm. Therefore, with traditional analytical techniques, there is a need to pretreat the sample to remove the interference molecules. In the present example no pretreatment is required as enzymes are used to specifically catalyze the AA molecules. The difference in absorption levels at 266 nm before and after target molecule consumption relates only to the AA initially present in the sample.

Ascorbate oxidase is an important oxidase enzyme existing in human plasma, or extracted from various plants, that can catalyze the oxidation of AA by oxygen to form dehydroascorbic acid (DA). There is a discrepancy in absorbance level of DA and AA.

An experiment to determine AA content in a vitamin C effervescent tablet was used as an example. The steps comprised immobilization of enzyme, calibrating the system 1, running the sample and analyzing the results.

Immobilization of enzyme:

1 $\mu$g/mL solution of ascorbate oxidase in 10 mM HEPEs buffer (pH 7.5) was continuously passed through a serpentine microfluidic channel at 20 $\mu$L/min for 1 hour. The microfluidic channel was then flushed by 10 mM HEPEs buffer (pH 7.5, adjusted with NaOH) at 20 $\mu$L/min for 20 min in order to remove excess and loosely bound enzymes. The channel was thereby coated with the enzyme 12 and a reaction flow channel 4 was formed on the chip of the system.

Calibration:

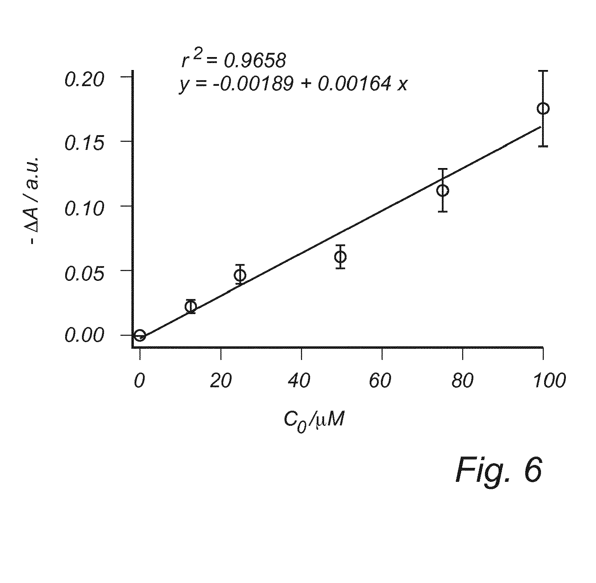

In order to calibrate the system, AA was dissolved in a 50 mM acetate buffer with a pH of 5.6 to be as standard solution. AA acted as the previously mentioned target molecule 16 with the buffer as matrix. A series of standard AA solutions (with known AA concentrations) in acetate buffer were directly injected into the UV/vis spectrophotometer to determine the UV-absorbance at 266 nm. The solutions were then injected into the flow reaction channel 4 of the chip 2. After oxidation of AA by contacting of the AA and the immobilized enzyme in the reaction flow channel 4, the solutions were analyzed again. The differences of absorbance at 266 nm before and after oxidation were calculated and plotted as a function of the original AA concentrations. The results are illustrated in Fig. 6. The calibration curve shows a good linearity for initial concentration of AA from 0 $\mu$M to 100 $\mu$M.

Experiments:

A vitamin C tablet was prepared in an aqueous solution to a concentration of 10 g/L. The solution was then diluted 400, 800 and 1600 times with 50 mM acetate buffer (pH 5.6). The diluted solution was passed through the reaction flow channel 4 at 5 $\mu$L/min separately, while determining the absorbance levels of each solution. The results were compared to the previously obtained calibration curve. The AA content in the vitamin C tablet in the present example was calculated to be 21.8(+/- 1.5) %. The result may be compared to the theoretical content of 22.2 (+/- 0.2) % indicated by the provider of the vitamin C tablet. The results show that it is practically feasible to analyze AA content in unknown samples by the enzymatic microfluidic sensor.

Tolerance against interfering molecules:

In order to verify the specificity of the system 1, caffeine was added to an AA containing solution as an artificial interference molecule. The summed absorbance ($A_{detected}$) can be expressed according to the Beer-Lambert law, as

$$A_{detected} = E_{AA,266nm}bc_{AA} + E_{caffeine,266nm}bc_{caffeine}$$

where $A_{detected}$ is the detected absorbance at 266 nm of the solution comprising AA and caffeine, $E_{AA,266nm}$ and $E_{caffeine,266nm}$ are the molar attenuation coefficients of AA and caffeine at 266 nm, respectively, b is the light path length (herein 1 cm), $c_{AA}$ and $c_{caffeine}$ are the molar concentration of AA and caffeine in the solution, respectively.

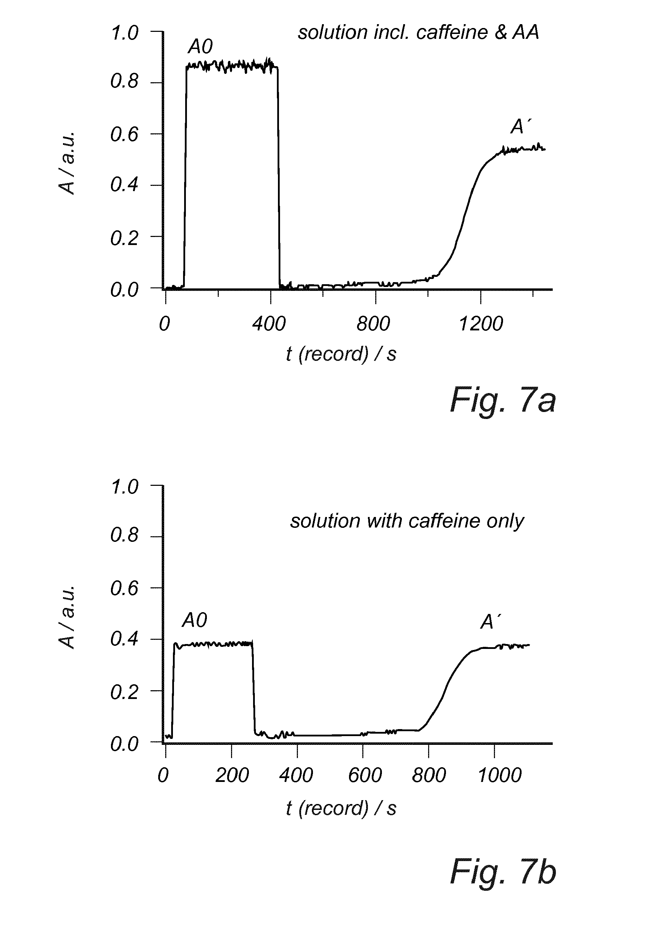

After the solution containing both AA and caffeine has passed through the reaction flow channel, a decline in absorbance at 266 nm can be detected. However, when a solution containing only caffeine was run through the same reaction flow channel, no significant absorbance change at 266 nm could be detected. The results are illustrated in Figs 7a and 7b, respectively. The ascorbate oxidase-loaded microfluidic sensor is therefore shown to be specific to the analysis of AA.

The person skilled in the art realizes that the present invention by no means is limited to the preferred embodiments described above. On the contrary, many modifications and variations are possible within the scope of the appended claims.

For example, the chip may be replaced with another structure comprising a reaction flow channel according to the first aspect, such as a capillary or tube, optionally attached to a chip or chip-like structure.

Additionally, variations to the disclosed embodiments can be understood and effected by the skilled person in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage.

## Claims

1.  A system for analysis of a target molecule in complex matrix sample, the system comprising:

    a chip (2) comprising a reaction flow channel (4) configured to receive the complex matrix sample, wherein the reaction flow channel is coated with an enzyme (12) configured to chemically change the target molecule;
    a flow generator (6), configured to provide a flow of the complex matrix sample through the reaction flow channel; and
    an absorbance detector (8) configured to determine the absorbance of the complex matrix sample after having passed through the reaction flow channel.

2.  The system according to claim 1, wherein the reaction flow channel is a microfluidic flow channel having a cross sectional area taken perpendicular to a flow direction of the complex matrix complex matrix sample through the reaction flow channel below 100 000 $\mu$m$^2$.

3.  The system according to claim 1 or 2, wherein the reaction flow channel is coated with the enzyme by physisorption.

4.  The system according to claim 1 or 2, wherein the reaction flow channel is coated with the enzyme by sol-gel encapsulation or by chemical immobilization.

5.  The system according to claim 1 or 2, wherein the reaction flow channel is chemically modified with the enzyme.

6. The system according to any one of claims 1-5, wherein the absorbance detector is an absorbance spectrophotometer.

7. The system according to claim 6, wherein the absorbance spectrophotometer is configured to determine absorbance in the visible and/or ultraviolet and/or near-infrared wavelength range of electromagnetic radiation.

8. A method for analysis of a target molecule in a complex matrix sample, the method comprising:

contacting (102) the complex matrix sample and an enzyme configured to chemically change the target molecule;
determining (104) a first absorbance of the complex matrix sample, which complex matrix sample has been subjected to the contacting according to a first degree;
determining (106) a second absorbance of the complex matrix sample, which complex matrix sample has been subjected to the contacting according to a second degree, wherein the second degree of contacting is larger than the first degree of contacting; and
comparing (108) the first absorbance and the second absorbance.

9. The method according to claim 8, wherein the first degree of contacting corresponds to no contacting.

10. The method according to claim 8 or 9, wherein the method further comprises coating a contacting surface with the enzyme prior to the contacting.

11. The method according to any one of claims 8-10, wherein the method further comprises quantifying the target molecule using the comparing.

12. The method according to anyone of claims 8-11, wherein the chemical change of the target molecules involves a consumption of the target molecule.

13. The method according to anyone of claims 8-12, wherein the first absorbance and the second absorbance are within the visible and/or ultraviolet and/or near-infrared wavelength range of electromagnetic radiation.

14. A method according to anyone of claims 8-13 using the system according to anyone of claims 1-7.

*Fig. 1*

*Fig. 2*

*Fig. 3*

*Fig. 4*

*100*

Contacting a complex matrix
sample with an enzyme — *102*

Determining a first
absorbance of the complex
matrix sample — *104*

Comparing first
absorbance and second
absorbance — *106*

Comparing first
absorbance and second
absorbance — *108*

*Fig. 5*

$r^2 = 0.9658$
$y = -0.00189 + 0.00164\,x$

*Fig. 6*

Fig. 7a

Fig. 7b

Europäisches
Patentamt

European
Patent Office

Office européen
des brevets

**EUROPEAN SEARCH REPORT**

Application Number

EP 15 18 1556

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MALENE S. THOMSEN ET AL: "Coated-wall microreactor for continuous biocatalytic transformations using immobilized enzymes", BIOTECHNOLOGY JOURNAL, vol. 4, no. 1, 10 July 2008 (2008-07-10), pages 98-107, XP055253403, DE ISSN: 1860-6768, DOI: 10.1002/biot.200800051 * page 99, right-hand column, line 37 - line 47 * * page 100, left-hand column, lines 26-31, 35-38 * * page 100, right-hand column, line 55 - page 101, left-hand column, line 7 * * page 101, left-hand column, lines 8-14, 19-23 * * page 101, left-hand column, line 23 - page 101, right-hand column, line 6 * * figures 1A, 1B * | 1-14 | INV. B01L3/00 |
| X | US 2003/231294 A1 (WARIAR RAMESH [US] ET AL) 18 December 2003 (2003-12-18) * paragraphs [0053] - [0064] * * figures 1, 2, 3 * | 1-14 | TECHNICAL FIELDS SEARCHED (IPC) B01L C12N G01N |
| A | US 6 020 207 A (LIU SU YI [US]) 1 February 2000 (2000-02-01) * the whole document * | 1-14 | |
| A | US 2013/081447 A1 (CARTER JERRY CHANCE [US] ET AL) 4 April 2013 (2013-04-04) * the whole document * | 1-14 | |

-/--

The present search report has been drawn up for all claims

| Place of search | Date of completion of the search | Examiner |
|---|---|---|
| The Hague | 29 February 2016 | Bischoff, Laura |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

                                                                 
& : member of the same patent family, corresponding
document

EPO FORM 1503 03.82 (P04C01)

Europäisches
Patentamt

European
Patent Office

Office européen
des brevets

# EUROPEAN SEARCH REPORT

Application Number

EP 15 18 1556

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2002/058273 A1 (SHIPWASH EDWARD [US]) 16 May 2002 (2002-05-16) * paragraphs [0174], [0187], [0349], [0354] * ----- | 1-14 | |
| A | US 2007/000781 A1 (DORDICK JONATHAN S [US] ET AL) 4 January 2007 (2007-01-04) * the whole document * ----- | 1-14 | |

TECHNICAL FIELDS
SEARCHED      (IPC)

The present search report has been drawn up for all claims

| Place of search | Date of completion of the search | Examiner |
|---|---|---|
| The Hague | 29 February 2016 | Bischoff, Laura |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

........................................................................

& : member of the same patent family, corresponding
     document

EPO FORM 1503 03.82 (P04C01)

## ANNEX TO THE EUROPEAN SEARCH REPORT
## ON EUROPEAN PATENT APPLICATION NO.

EP 15 18 1556

This annex lists the patent family members relating to the patent documents cited in the above-mentioned European search report.
The members are as contained in the European Patent Office EDP file on
The European Patent Office is in no way liable for these particulars which are merely given for the purpose of information.

29-02-2016

| Patent document cited in search report | | Publication date | Patent family member(s) | | Publication date |
|---|---|---|---|---|---|
| US 2003231294 | A1 | 18-12-2003 | AU 2003237275 A1<br>US 2003231294 A1<br>WO 03106981 A1 | | 31-12-2003<br>18-12-2003<br>24-12-2003 |
| US 6020207 | A | 01-02-2000 | NONE | | |
| US 2013081447 | A1 | 04-04-2013 | NONE | | |
| US 2002058273 | A1 | 16-05-2002 | AU 8485601 A<br>CA 2314398 A1<br>US 2002058273 A1<br>US 2005164264 A1<br>WO 0212855 A2 | | 18-02-2002<br>10-02-2002<br>16-05-2002<br>28-07-2005<br>14-02-2002 |
| US 2007000781 | A1 | 04-01-2007 | US 2007000781 A1<br>US 2007108057 A1 | | 04-01-2007<br>17-05-2007 |

EPO FORM P0459

For more details about this annex : see Official Journal of the European Patent Office, No. 12/82